Europäisches Patentamt

European Patent Office

Office européen des brevets

(11) Publication number : **0 520 779 A2**

## (12) EUROPEAN PATENT APPLICATION

(21) Application number : 92305821.8

(22) Date of filing : 24.06.92

(51) Int. Cl.⁵ : **C07C 5/48,** C07C 15/46

(30) Priority : 28.06.91 US 721764

(43) Date of publication of application :
30.12.92 Bulletin 92/53

(84) Designated Contracting States :
AT BE CH DE DK ES FR GB GR IT LI LU MC NL
PT SE

(71) Applicant : **ROHM AND HAAS COMPANY**
**Independence Mall West**
**Philadelphia Pennsylvania 19105 (US)**

(71) Applicant : **Petrosius, Steven**
**929 NE 12th Avenue**
**Gainesville, Florida 32601 (US)**

(71) Applicant : **Grunewald, Gerald**
**4705 Burns Court**
**Orange, Texas 77630 (US)**

(71) Applicant : **KRZYSZTOF, Jurczyk**
**1216 SW 2nd Avenue, Apt.145**
**Gainesville, Florida 32601-6143 (US)**

(71) Applicant : **Drago, Russell**
**2281 NW 24th Avenue**
**Gainesville, Florida 32605 (US)**

(72) Inventor : **Brendley, William Harry, Jr.**
**2450 Exton Road**
**Hatboro, Pennsylvania 19040 (US)**
Inventor : **Petrosius, Steven**
**929 NE 12th Avenue**
**Gainesville, Florida 32601 (US)**
Inventor : **Krzysztof, Jurczyk**
**1216 SW 2nd Avenue, Apt. 145**
**Gainesville, Florida 32601-6143 (US)**
Inventor : **Grunewald, Gerald**
**4705 Burns Court**
**Orange, Texas 77630 (US)**
Inventor : **Drago, Russell Stephen**
**2281 NW 24th Avenue**
**Gainesville, Florida 32605 (US)**

(74) Representative : **Tanner, James Percival et al**
**ROHM AND HAAS (UK) LTD. European**
**Operations Patent Department Lennig House**
**2 Mason's Avenue**
**Croydon CR9 3NB (GB)**

(54) Oxidative dehydrogenation of hydrocarbons with a carbon-containing catalyst.

(57) A method is disclosed for the oxidative de-
hydrogenation of hydrocarbon compounds
such as alkylated aromatics, alkanes, alkenes,
and oxygenates such as alcohols. The method
comprises contacting the hydrocarbon com-
pound with oxygen in the presence of oxidative
dehydrogenation catalyst. The catalyst exhibits
good selectivity to desired products and has
sufficient activity to maintain low reaction tem-
peratures. Further, there are disclosed catal-
ysts, doped with a redoxactive transition metal
moiety, which exhibit increased activity and
selectivity.

EP 0 520 779 A2

The present invention is concerned with a method for oxidative dehydrogenation of hydrocarbon compounds. In particular, the present invention is concerned with a method for oxidative dehydrogenation of hydrocarbon compounds in the presence of carbonaceous catalysts.

Dehydrogenation of hydrocarbons is a commercially important process. For example, dehydrogenation of ethylbenzene to styrene provides a commercially significant reaction product. However, direct dehydrogenation is an endothermic reaction limited by equilibrium. Therefore, typically, yield does not exceed 50 percent. Further, even though this reaction is carried out in the presence of a promoted iron oxide catalyst, reactor temperatures typically exceed 600°C.

These limitations of the direct dehydrogenation method have resulted in the investigation of other methods for obtaining dehydrogenated products. In particular, oxidative dehydrogenation has been proposed as a method which may obviate the high temperature requirement. A significant advantage of oxidative dehydrogenation lies in the completeness of conversion which , theoretically, can be achieved because the formation of water as a byproduct of the reaction makes the reaction exergonic. Therefore, under typical reaction conditions, the exothermic heat of reaction sustains continued reaction.

A number of materials have been proposed as catalysts for the oxidative dehydrogenation reaction. Promoted inorganic oxide catalysts proposed include tin- and phosphorus-containing oxides ($SnO_2$-$P_2O_5$ ) and zirconium- and phosphorus-containing oxides ($ZrO_2$-$P_2O_5$). Cerium pyrophosphate also has been utilized to catalyze the reaction. These catalysts exhibit at least about 80 percent selectivity with 30 to about 75 percent conversion for the oxidative dehydrogenation of ethylbenzene to styrene. However, none of these oxide catalysts enables lowering the reaction temperature below about 550°C.

The article Oxidative Dehydrogenation of Ethylbenzene to Styrene Over Carbon-Based Catalysts, J. Molec. Catal. 58 (1990), at 227-233, describes oxidative dehydrogenation of ethylbenzene utilizing inter alia various carbonaceous substances as catalyst. The carbonaceous substances include 3 types of pyrolyzed polyacrylonitrile (PPAN), each of which has a specific surface area less than about 50 m2 / gram, activated carbon having a specific surface area of about 800 m2/ gram, and a carbon molecular sieve material having an extraordinarily high specific surface area. Only the carbon molecular sieve material yielded a conversion exceeding 30 percent.

Efforts to lower the temperature at which the oxidative dehydrogenation reaction is carried out eventually resulted in the use of carbon molecular sieves. Such material has a uniform surface structure, an extremely high specific surface area, and a very narrow pore size range. Further, it is believed that the surface structure of the material is particularly suited for catalyzing the oxidative dehydrogenation process. However, no definitive conclusion was drawn therein regarding the characteristics of the catalyst which result in the extraordinarily high activity (conversion) and selectivity (yield of desired product).

Data in the Oxidative Dehydrogenation article illustrates that high specific surface area alone does not ensure that satisfactory conversion will be achieved. As illustrated therein, activated carbon having a specific surface area an order of magnitude higher than the pyrolyzed polyacrylonitrile (PPAN) catalyst utilized therein exhibited activity (conversion) essentially equal to that of the PPAN.

Oxidative dehydrogenation of aliphatic nitriles catalyzed with carbonaceous catalysts is disclosed in US-A-4,948,910. This patent discloses that the preferred catalyst has three distinct sets of pores, each having a different average size, and expresses a preference for catalyst having a specified minimum pore volume of the sub-micropores, defined therein as those pores having an average critical dimension of less than about 7 angstroms. This patent suggests that the sub-micropores control product selectivity, and teaches that catalyst having any specific surface area is satisfactory, provided that the catalyst exhibits oxidative dehydrogenation activity.

The disclosure of US-A-4,948,910 is limited to dehydrogenation of aliphatic nitriles. Although the role of the nitrile moiety has not been determined, the nitrile moiety is expected to be a good donor moiety for binding the nitrile-containing molecule to a catalyst. The nitrile moiety also may play an important part in stabilizing intermediates in the oxidative dehydrogenation method.

Selected molecular carbon sieve material has been-utilized to catalyze oxidative dehydrogenation of alkyl aromatic compounds, particularly ethylbenzene and ethyltoluene. US-A-4,652,690 discloses that molecular carbon sieve material having a pore size of 5-7 angstroms is an active oxidative dehydrogenation catalyst for formation of styrene from ethylbenzene.

According to the present invention there is provided a method for oxidative dehydrogenation of a hydrocarbon compound to produce a more unsaturated product, said method comprising:

reacting the hydrocarbon compound and oxygen in the presence of oxidative dehydrogenation catalyst under conditions sufficient to remove hydrogen atoms from the hydrocarbon compound and form a more unsaturated product and water, said catalyst comprising carbonaceous material selected from:

(a) carbonaceous material having a selected specific surface area and a selected pore size distribution, the carbonaceous material being selected from polymeric carbons prepared by pyrolysis of resinous polymers, polymeric carbons prepared by pyrolysis of petroleum residues, polymeric carbons prepared by pyrolysis of other polymeric carbon precursors, and blends thereof;

(b) carbonaceous material doped with a redox-active transition metal moiety and selected from polymeric carbons prepared by pyrolysis of resinous polymers, polymeric carbons prepared by pyrolysis of petroleum residues, polymeric carbons prepared by pyrolysis of other polymeric carbon precursors, and blends thereof; and

(c) blends thereof.

Preferably, the catalyst used in the present invention is selected from carbonaceous material having a selected specific surface area and a selected pore size distribution selected from polymeric carbons prepared by pyrolysis of resinous polymers, carbonaceous material doped with a redox-active transition metal moiety and selected from polymeric carbons prepared by pyrolysis of resinous polymers, and blends thereof.

The present invention also provides a catalyst for the oxidative dehydrogenation of hydrocarbon compounds comprising carbonaceous material doped with a redox-active transition metal moiety and selected from polymeric carbons prepared by pyrolysis of resinous polymers, polymeric carbons prepared by pyrolysis of petroleum residues, polymeric carbons prepared by pyrolysis of other polymeric carbon precursors, and blends thereof.

The present invention is directed to a method for oxidative dehydrogenation of hydrocarbon compounds. Hydrocarbon compounds, including alkylated aromatics, alkanes, alkenes, oxygenates such as alcohols, and blends thereof, are contacted with oxygen in the presence of oxidative dehydrogenation catalyst to produce a more unsaturated product. The catalyst exhibits good selectivity to desired products and has sufficient activity to maintain low reaction temperatures. Catalyst doped with redox-active transition metal moiety exhibits increased activity and selectivity. Further, catalyst doped with a redox-active transition metal moiety which also catalyzes reaction of material which is a by-product of the oxidative dehydrogenation reaction and is deposited on the catalyst during the course of that reaction exhibits reduced tendency to agglomerate.

This invention is based on the finding that carbonaceous catalysts having selected specific surface area and selected distribution of pores having diameters within selected ranges exhibit extraordinary activity in catalyzing the oxidative dehydrogenation of hydrocarbon compounds such as alkylated aromatics, alkanes, alkenes, and oxygenates. Not only are these catalysts especially active, but also they exhibit excellent selectivity to the desired product. It also has been discovered that doping such catalysts with a redox-active transition metal moiety increases both the activity and selectivity of the resulting catalyst. Further, doping such catalysts with a redox-active transition metal moiety which catalyzes reaction of material which is a by-product of the oxidative dehydrogenation reaction and is deposited on the catalyst during the course of that reaction reduces the tendency for the catalyst to agglomerate.

The invention is directed to a method for oxidatively dehydrogenating hydrocarbon compounds. Throughout the specification and claims, the phrase 'hydrocarbon compound' and the word 'hydrocarbon', when used to describe a reactant to be dehydrogenated in accordance with the method of the invention, means any compound susceptible of dehydrogenation which consists essentially of carbon and hydrogen, and oxygenated forms thereof. Therefore, compounds containing nitrogen and other strong donor groups, such as amines, sulfides, phosphides, and nitriles, are not within the scope of the invention. Rather, the invention is directed to a method for oxidatively dehydrogenating hydrocarbon compounds such as alkylated aromatics, alkanes, alkenes, and oxygenates.

Oxidative dehydrogenation of alkylated aromatic, alkane, alkene, and oxygenated hydrocarbon reactants results in the removal from the hydrocarbon reactant of at least two atoms of hydrogen and the formation of one molecule of water. The resulting product is said to be more unsaturated, or less saturated, than the feed from which the product was obtained. Skilled practitioners recognise that hydrogen atoms are removed in pairs, i.e., one hydrogen atom from each of two adjacent carbon atoms, and that more than one pair of hydrogen atoms may be removed from a molecule. For example, butane can be oxidatively dehydrogenated to form not only 1-butene but also 1,3-butadiene.

In accordance with the method of the invention, hydrocarbon reactant and oxygen are delivered to a bed of catalyst at appropriate temperature and pressure. The reactant and oxygen are brought into contact in the presence of catalyst for a period sufficient to achieve oxidative dehydrogenation of the reactant and to yield a desired unsaturated hydrocarbon. In the case where the reactant is not saturated, the method provides a product which has a greater degree of unsaturation.

Hydrocarbons of any molecular weight may be treated in accordance with the method of the invention. Typically, however, it is the lower molecular weight hydrocarbons of each type which typically are dehydrogenated in accordance with the method of the invention. For example, alkanes, alkenes, oxygenates, and the alkyl moieties of alkylated aromatics, dehydrogenated in accordance with this method, typically have up to about 6 carbon atoms. Preferably, the hydrocarbon compound is selected from alkylated aromatics wherein the alkyl group has up to about 6 carbon atoms; straight chain, branched, and cyclic alkanes, alkenes, and oxygenates having up to about 6 carbon atoms; and blends thereof.

Examples of alkylated aromatics which can be treated in accordance with the method of the invention include ethylbenzene and diethylbenzene. The desired product resulting from oxidative dehydrogenation of ethylbenzene in accordance with the method of the invention is styrene. The desired products of the dehydrogenation of 1,4-diethylbenzene include p-ethylstryrene and divinylbenzene. Similarly, the desired product of oxidative dehydrogenation of an alkane such as butane is 1-butene, and 1,3-butadiene is the desired result of the dehydrogenation of 1-butene. The products of the oxidative dehydrogenation of ethanol (an oxygenate) include acetaldehyde and ethyl acetate, with a minor fraction of diethyl ether. It will be apparent to the skilled practitioner that other unsaturated compounds will be obtained by oxidative dehydrogenation of other starting materials.

These unsaturated products have uses known to skilled practitioners. For example, styrene can be polymerized, or can be copolymerized with, for example, butadiene (itself a product of the method of the invention), isoprene, and acrylonitrile. Other unsaturated products of the method of the invention have well-known uses.

The reactants are delivered to the catalyst bed in any suitable form. Typically, the lower molecular weight reactants, particularly the alkanes and alkenes, are in the vapour state both at ambient conditions and at the conditions under which reaction takes place. Other reactants, such as ethanol, may be liquid at ambient, but vapour at reaction conditions. Still other hydrocarbon reactants may remain liquid throughout. Preferably, however, the reactants are in the vapour phase throughout the reaction zone.

The oxygen required in the oxidative dehydrogenation reaction is provided in any form in which the oxygen molecules are available for reaction with the hydrocarbon reactant. Oxygen typically is supplied in gaseous form. Suitable sources of oxygen include air, ozone, purified oxygen, and mixtures thereof. Utilization of air as the oxygen source carries with it the advantage of low cost and ease of availability.

Preferably, the oxygen concentration in the oxygen source is limited to less than about 50 percent, more preferably less than about 30 percent, by combination of the oxygen with a non-reactive diluent. Introduction of non-reactive diluent provides a way to control the exothermic reaction. The diluent carries some of the heat of reaction out of the reaction zone, thus helping control the temperature therein. Suitable diluents include water vapour (steam), the noble (or inert) gases, including helium and argon, carbon dioxide, and nitrogen.

Any concentration of oxygen in the oxygen source sufficient to provide the oxygen required for the reaction is suitable for use in the method of the invention. Skilled practitioners recognize that use of pure oxygen minimizes the chance of contamination of the product by introduction of impurities and reduction of yield by side reactions. However, such precautions typically are not necessary, and air is a preferred oxygen source. Diluted oxygen also is a preferred oxygen source, with steam a preferred diluent.

Any quantity of oxygen in the reactant mixture sufficient to promote the oxidative dehydrogenation of the reactant is suitable for use in the method of the invention. Preferably, between about 0.1 and about 5 moles of oxygen are present in the reaction mixture per mole of reactant. More preferably, the molar ratio of oxygen to reactant is between about 0.3:1 and about 4:1. Most preferably, a molar ratio of oxygen to reactant is between about 0.5:1 and about 3:1. A molar ratio of oxygen to reactant of less than about 0.1:1 may not provide sufficient oxygen to carry out the oxidative dehydrogenation reaction. Oxygen:reactant molar ratios above 5:1 may cause loss of selectivity to desired unsaturated product through oxidation of the desired product or of the hydrocarbon reactant. Further, high oxygen concentrations in the mixture of reactants may not provide sufficient removal of the exothermic heat of reaction. Skilled practitioners recognize that explosive mixtures of hydrocarbon reactant and oxygen are not recommended.

Any temperature at which the oxidative dehydrogenation reaction proceeds is suitable for use in the method of the invention. Typically, a temperature of at least about 200°C is utilized. The reaction rate at a temperature below this temperature may be slow or the selectivity low. Further, a temperature of less than about 550°C typically is utilized. At a temperature higher than about 550°C, the exothermic reaction may become difficult to control. The selectivity also may decrease at a temperature above about 550°C because oxidation of the reactant itself may become significant. Therefore, the temperature preferably is maintained between about 200°C and about 550°C, more preferably between about 250°C and about 500°C, and most preferably between about 300°C and about 400°C.

The pressure at which the reaction is carried out is selected to ensure that the oxidative dehydrogenation proceeds at an acceptable rate and produces desired unsaturated product. Typically, an absolute pressure of between about $6.895 \times 10^3$ and $10.34 \times 10^6$ Pa absolute (1 and 1500 psia) is utilized. Preferably, the pressure is maintained between about $34.48 \times 10^3$ and $1.03 \times 10^6$ Pa absolute (5 and about 150 psia), more preferably, between about $51.71 \times 10^3$ and $344.75 \times 10^3$ Pa absolute (7.5 and 50 psia).

Feedrate of hydrocarbon compound reactant is established to provide a sufficient period of contact between catalyst and reactants. Skilled practitioners recognize that the time required to achieve the desired oxidative dehydrogenation is related not only to the identity of the hydrocarbon compound undergoing oxidative dehydrogenation, but also upon the operating conditions, including temperature and pressure. The liquid space velocity (LSV), i.e., the liquid volume of reactant passed over a volume of catalyst per unit time, typically is between about 0.005 hr$^{-1}$ and about 50 hr$^{-1}$. More typically, the LSV is between about 0.01 hr$^{-1}$ and about 10 hr$^{-1}$, and most typically is between about 0.1 hr$^{-1}$ and about 5 hr$^{-1}$.

Catalysts utilized in the method of the invention are carbonaceous materials having a selected specific surface area and a selected distribution of pores having diameters within selected ranges. It has been found that carbonaceous materials having the selected specific surface area and selected pore size distribution effectively catalyze oxidative dehydrogenation of hydrocarbons. It has further been found that the activity and selectivity of these and other carbonaceous materials are enhanced with redox-active transition metal moieties. Although we do not wish to be bound by theory, it is believed that the activity of the catalyst may be related not only to the specific surface area and pore size distribution, but also to the redox activity of active sites within the pores.

As used herein, the selected pore size distribution refers to a distribution of pores having diameters within selected ranges, and requires that the catalyst have pores within each of three distinct sets of pores of differing average diameter, and that the specific pore volume within each set be at least about 0.05 ml/g. These sets are known as macropores, mesopores, and micropores, in accordance with IUPAC nomenclature. The specific volume of pores in each set is known as macroporosity, mesoporosity, and microporosity, respectively. The set which comprises the large pores, called macropores, encompasses those pores having a diameter greater than 500 angstroms. The mesopore set comprises those pores having diameters between about 20 and about 500 angstroms. The smallest pores, called micropores, have diameters up to about 20 angstroms.

The selected pore size distribution for undoped catalysts suitable for use in the method of the invention requires at least about 0.05 ml/g each macroporosity, mesoporosity, and microporosity. Preferably, each porosity exceeds 0.10 ml/g, and the microporosity and macroporosity exceeds the mesoporosity. Most preferably, the microporosity exceeds 0.2 ml/g. Especially preferred is a microporosity exceeding 0.3 ml/g.

Catalyst suitable for use in the method of the invention without doping may be made by any method and from any starting material which produces the desired specific surface area and desired pore size distribution. In particular, carbonaceous material is selected from the group consisting of polymeric carbons prepared by pyrolysis of resinous polymers, polymeric carbons prepared by pyrolysis of petroleum residues, and polymeric carbons prepared by pyrolysis of other polymeric carbon precursors, such as lignin, vegetable shells (coconut shells) and other fibrous organic materials, and other carbon-containing materials, such as coal or animal bones, and blends thereof. Carbonaceous material suitable for use in the method of the invention thus is significantly different from other forms of carbons from such materials because the selected specific surface area and selected pore size distribution are not found in, for example, the PPAN or activated carbon materials described in the article Oxidative Dehydrogenation. In particular, carbon molecular sieve material, such as the product available as AX21 (available from Anderson Development Company), typically is essentially devoid of macroporosity, and therefore are not within the scope of the invention with regard to undoped catalyst.

Further, it has been found that carbons doped with a redox-active transition metal moiety need not have both the selected specific surface area and the selected pore size distribution. For example, carbon molecular sieve material, such as AX-21, is a suitable doped catalyst.

Preferred catalyst is selected from the group consisting of polymeric carbons prepared by pyrolysis or resinous polymers, polymeric carbons prepared by the pyrolysis of a resinous polymer or by pyrolysis of petroleum residues and doped with a redox-active transition metal moiety, and blends thereof. Particularly preferred catalysts are those doped with a redox-active transition metal moiety which acts as a catalyst for decomposition of by-products of the oxidative dehydrogenation reaction which are deposited on the catalyst.

Catalysts prepared by the pyrolysis of resinous polymers and carbonaceous materials prepared by pyrolysis of petroleum residues are known in the art. Pyrolyzed resinous catalysts and their method of preparation are described in US-A-4,040,990 and US-A-4,839,331, which are incorporated herein by reference. As described in US-A-4,040,990, these carbons are partially pyrolyzed particles preferably in the form of hard beads or spheres. They are produced by the controlled decomposition of a synthetic polymer. In particular, one such polymer is a sulfonated, macroreticular divinylbenzene crosslinked polystyrene. One such polymer described in US-A-4,839,331 is a polysulfonated, macroreticular divinylbenzene crosslinked polystyrene.

The pyrolysis, as described in US-A4,040,990, is generally conducted in an inert atmosphere comprising, for example, helium, argon, or nitrogen. Preferably, the polymer is heated rapidly to a maximum temperature in the range from about 300°C to about 900°C, heated at the maximum temperature for a period of up to about 20 minutes, and cooled to room temperature before being exposed to air. For the purposes of this invention, maximum temperatures of up to about 1200°C are also suitable, and longer heating times are not deleterious.

Any of the many synthetic polymers, disclosed in US-A-4,040,990 and US-A-4,839,331, each of which is incorporated herein by reference, can be employed in producing the catalyst for the process of this invention. Preferred are polymers derived from aliphatic and aromatic materials which are ethylenically unsaturated. Preferably, the polymer is cross-linked, because cross-linking stabilizes the polymer thermally and leads to greater carbon yields. Preferably also, the polymer contains a carbon-fixing moiety, such as a cation, anion, strong base, weak base, sulfonic acid, carboxylic acid, halogen, or alkylamine moiety. The more preferred polymers include polyvinylidene chloride and macroreticular ion-exchange resins derived from aliphatic and aromatic materials which are ethylenically unsaturated. Most preferably, the polymer is a polystyrene divinylbenzene sulfonic acid ion-exchange resin, which also may be characterized as a sulfonated, macroreticular divinylbenzene cross-linked polystyrene polymer.

In such carbonaceous materials prepared by pyrolysis of a resinous polymer, the exact pore size distribution depends inter alia on the temperature of pyrolysis. In addition to controlling pore diameter, the pyrolysis temperature also controls total pore volumes. Generally, as the pyrolysis temperature increases, mesoporosity decreases and microporosity increases. However, at pyrolysis temperatures about 900°C, the micropore volume may be low.

Catalyst used in this invention has a specific surface area sufficient to make the catalyst sufficiently active in the oxidative dehydrogenation reaction. For example, the catalyst may have a specific swface area of at least about 250 $m^2$/gram. Undoped catalysts of the invention typically have a specific surface area of between about 250 and 1500 $m^2$/gram, preferably between about 300 and about 1250 $m^2$/gram, and more preferably between about 400 and 1100 $m^2$/gram. Doped catalysts which do not have the selected pore size distribution typically have higher specific surface areas. For example, carbon molecular sieve material typically has a specific surface area of at least about 2000 $m^2$/gram. Typically, higher specific surface area yields greater activity. The surface area is measured by the Brunauer-Emmett-Teller (BET) method. The BET method is described by R.B. Anderson, in Experimental Methods in Catalytic Research, Academic Press, 1968, pp. 48-66.

As described in US-A-4,839,331, carbonaceous materials also can be activated by heating in an activating atmosphere. Suitable activating gases include oxygen, steam, water, ammonium, carbon monoxide, and carbon dioxide. Such activated materials are preferred catalysts in the practice of the method of the invention.

Catalysts which are carbonaceous material produced in accordance with the method of US-A-4,040990 and US-A-4,839,331 are available from the Rohm and Haas Company under the tradename Ambersorb carbonaceous adsorbent ("Ambersorb is a trademark of Rohm and Haas Company). The Ambersorb carbonaceous adsorbents are hard, non-dusting spheres with selected specific surface areas and selected pore distribution, as those terms are defined herein. In particular, Ambersorb carbonaceous adsorbent sold under the identifications 348F, 563, 564, 572, and 575 are particularly preferred in the practice of the method of the invention.

Ambersorb carbonaceous adsorbent is prepared in either activated or unactivated form. Unactivated material is hydrophobic and non-polar, and exhibits a pore size distribution having less microporosity than activated material. For example, Ambersorb carbonaceous adsorbent 563 and 564 are unactivated, and have less than about 0.3 ml/g microporosity, as determined by nitrogen porosimetry on a Micromeritics 2400. Activated Ambersorb carbonaceous adsorbent, such as the product identified 572 or 575, is significantly less hydrophobic than unactivated material and exhibits polar characteristics (contributed by the oxygen-containing surface functionality imparted by activation). Importantly, the microporosity exceeds 0.30 ml/g. Also, the specific surface area of activated material typically is higher than that of unactivated material.

Catalyst produced by pyrolyzing resinous materials also includes pyrolyzed polyacrylonitrile (PPAN). PPAN can be prepared in accordance with any method which yields material having the desired specific surface area and pore distributions. In particular, the polyacrylonitrile (PAN) preferably is cross-linked to provide superior pyrolyzed material. In one suitable two-step process, preliminary oxidation is carried out by heating PAN in air for up to about 24 hours at a temperature of between about 150 and 300°C. In the second step, the pre-oxidized material is carbonized by heating to a temperature between 500 and 1200°C in an inert atmosphere. If desired, a third (activation) step may be carried out. The material is heated to a temperature above about 600°C in an oxidizing atmosphere. Activation increases the specific surface area of the material, thus increasing its activity.

Carbonaceous materials produced by pyrolyzing petroleum residues also are known in the art. Both the materials and a method for their preparation are set forth in US-A-4,082,694, which is incorporated herein by reference. In particular, such materials are available from Anderson Development Company under the name of Carbon Molecular Sieves (CMS). Anderson's CMS product identified AX21 is a particularly useful carbonaceous material which yields a suitable oxidative dehydrogenation catalyst when doped.

Preferably, the carbonaceous materials and catalysts, e.g. having the selected specific surface area and selected pore size distribution, are doped with redox active transition metal moieties which can themselves, or in combination with other materials, function as a Bronsted acid or as a Lewis acid. Suitable dopants are selected from the group consisting of $CrO_3$, $MoO_3$, $MnO_2$, $V_2O_5$, $Ce(NO_3)_3$, $TiO_2$, and mixtures thereof. Preferred dopants are selected from the group consisting of $CrO_3$, $MoO_3$ and blends thereof.

The concentration of dopant utilized in the invention is that quantity sufficient to catalyze the combustion of by-product deposits. Therefore, the concentration of dopant on carbonaceous material is at least about 0.00001 gmol/gram of carbonaceous material. Typically, the dopant concentration does not exceed about 0.1 gmol/gram of carbonaceous material. Preferably, the dopant concentration is between about 0.00003 and about 0.01 gmol/gram, more preferably between about 0.00005 and about 0.005gmol/gram.

During the oxidative dehydrogenation reaction, by-products of the reaction may be deposited on the catalyst. Although we do not wish to be bound by theory, it is believed that these deposits cause the individual catalyst particles to form agglomerates with other particles. Agglomeration reduces yield of desired product by decreasing both catalytic activity and selectivity. It has been found that dopants catalyze the combustion of these deposits to carbon dioxide under the operating conditions in the reactor. Thus, use of these dopants reduces the tendency of the catalyst to agglomerate.

Thus, dopants which are more preferred catalyze both the desired oxidative dehydrogenation reactions and the combustion of undesired by-product deposits to carbon dioxide. More preferred dopants include $CrO_3$, $MoO_3$, $MnO_2$, $V_2O_5$, $Ce(NO_3)_3$, $KMnO_4$ and NaOH, $TiO_2$, and mixtures thereof, especially $CrO_3$, $MnO_2$, $V_2O_5$, $Ce(NO_3)_3$, $KMnO_4$ /NaOH, and blends thereof. It is believed that manganese hydroxide is formed when the preferred combination of $KMnO_4$ and NaOH is utilized.

Accordingly, the present invention further provides a method for preventing agglomeration caused by deposition of byproducts of oxidative dehydrogenation of hydrocarbon compounds on oxidative dehydrogenation catalyst comprising carbonaceous material selected from:

(a) carbonaceous material having a selected specific surface area and a selected pore size distribution selected from polymeric carbons prepared by pyrolysis of resinous polymers, polymeric carbons prepared by pyrolysis of petroleum residues, polymeric carbons prepared by pyrolysis of other polymeric carbon precursors, and blends thereof;

(b) carbonaceous material doped with a redox-active transition metal moiety and selected from polymeric carbons prepared by pyrolysis of resinous polymers, polymeric carbons prepared by pyrolysis of petroleum residues, polymeric carbons prepared by pyrolysis of other polymeric carbon precursors, and blends therefore; and

(c) blends thereof;

said method comprising doping the catalyst with a moiety which catalyzes the combustion of the deposited byproducts to carbon dioxide.

Doping may be carried out by any suitable method. Typically, solvent impregnation, vapour deposition, or the molten salt method are used. Solvent impregnation is preferred for impregnation of most soluble metal compounds on non-graphitizable carbons, such as the Ambersorb carbonaceous material. Typically, dopant is dissolved in a quantity of solvent sufficient to completely occupy the interior pore volume of the carbonaceous material. Then, metal-containing solution is added. Solvent then is removed by heating the material, often under vacuum. For 'soft' carbons, excess solvent is utilized under reflux conditions until uniform dispersion is obtained. Then, excess solvent is removed by rotary evaporation before heat/vacuum drying. Vapour deposition, i.e. contacting the carbonaceous material with gaseous meta-containing compound, also is suitable.

The oxidative dehydrogenation reaction may be carried out in any suitable reactor. Skilled practitioners will be able to select suitable materials of construction. Although a batch-type reactor can be utilized, a flow reactor is preferred. Typically, the reactor bed will comprise not only catalyst but also support materials at each end. Support material serves not only to support the catalyst bed physically, but also to both mix and modulate the temperature of the materials flowing into the reactor. Vertical and horizontal flow patterns are suitable.

Typically, gaseous reactants are mixed before contacting the catalyst. Liquid products are collected in a trap near the catalyst zone; gaseous products are cooled, collected, and separated as required, for example, by fractional distillation.

Skilled practitioners recognise that some hydrocarbon compounds are more difficultly dehydrogenated than others. For example, isobutane is difficultly dehydrogenated, and dehydrogenation of 1,4-diethylbenzene to divinylbenzene is difficult. Similarly, a catalyst may be a particularly active oxidative dehydrogenation catalyst with respect to a hydrocarbon compound yet be a relatively inactive oxidative dehydrogenation catalyst with respect to another hydrocarbon compound. Thus, although a catalyst which is particularly active with respect to a hydrocarbon compound may not exhibit significant activity with respect to all hydrocarbon compounds, that catalyst is intended to be within the scope of the invention as defined by the claims.

The following Examples are presented to illustrate preferred embodiments of the present invention.

## EXAMPLES

## Example 1

### Part 1 - Oxidative Dehydrogenation of Ethylbenzene

In a vertical fixed-bed reactor, 0.5 grams of Ambersorb 563 carbonaceous adsorbent formed a catalyst bed having a 17 mm bed depth. Glass beads were placed on top of the catalyst bed and heated to approximately the same temperature as the catalyst bed to ensure that the incoming mixture of reactants was properly mixed, vaporized, and heated to the desired temperature.

Ethylbenzene and air were fed into the top of the reactor, i.e., into the top of the glass beads, and passed through the catalyst bed. The feedrate of the ethylbenzene was 0.2 ml of liquid per hour, while the air flow rate was 300 ml/hour. Oxidative dehydrogenation of the ethylbenzene was carried out with the pressure in the reactor maintained at $103.43 \times 10^3$ Pa absolute (15 psia); the temperature of the catalyst was 360°C. Reaction was continued for 15 hours.

The effluent gas stream was analyzed by both FID and TCD gas chromatographic analyses, which indicated that 38.1 percent of the ethylbenzene had been converted, with a selectivity to styrene of 96.5 percent. Both activity and selectivity were confirmed by analysis of the effluent after condensing the effluent at 0°C.

### Part 2 - Absence of Oxygen

After the 15 hour period of oxidative dehydrogenation, the flows of air and liquid ethylbenzene were stopped. Nitrogen at a flowrate of 300 ml/hour was used to purge remaining oxygen from the reactor. After confirming that the reactor was devoid of oxygen, the ethylbenzene flowrate was re-established at a rate of 0.2 ml/hour. Oxygen was excluded from the reactor for 10 hours. During this period when no oxygen was fed to the reactor, the conversion of ethylbenzene was 1.9 percent, with selectivity to styrene of 94.8 percent.

### Part 3 - Confirmation of Catalytic Activity

Air was substituted for nitrogen and oxidative dehydrogenation of ethylbenzene was carried out for a second 15-hour period. Analysis of the reactor effluent indicated that conversion of the ethylbenzene was 34.5 percent, with 96.1 percent selectivity to styrene.

## Example 2

The method of each part of Example 1 was repeated, with Ambersorb 564 carbonaceous adsorbent utilized as catalyst. During the initial period of oxidative dehydrogenation of Part 1, the conversion of the ethylbenzene was 37.1 percent, with a selectivity of styrene of 98.2 percent. After elimination of oxygen from the reactor with nitrogen, as in Part 2, conversion of ethylbenzene was reduced to only 3.0 percent, with 55.4 percent selectivity to styrene. Thereafter, when oxidative dehydrogenation conditions again were established, 16.0 percent conversion of ethylbenzene, with selectivity to styrene of 99.3 percent, was achieved.

## Example 3

The method of each part of Example 1 was repeated, with Ambersorb 563 carbonaceous adsorbent utilized as catalyst. The height of the catalyst bed, which contained 0.5 grams of catalyst, was 22 mm. During the initial period of oxidative dehydrogenation in accordance with the method set forth in Part 1, the conversion of the ethylbenzene was 18.5 percent, with selectivity to styrene of 92.4 percent. After elimination of oxygen from the reactor with nitrogen, as in Part 2, conversion of ethylbenzene was reduced to only 3.5 percent, with 95.2 percent selectivity to styrene. Thereafter, when oxidative dehydrogenation conditions were again established, 54.9 percent conversion of ethylbenzene, with selectivity to styrene of 98.8 percent, was achieved.

## Example 4

The method of each part of Example 1 was repeated, with Ambersorb 575 carbonaceous adsorbent utilized as catalyst. The height of the 0.5-gram catalyst bed was 21 mm. During the initial period of oxidative dehydrogenation of Part 1, the conversion of the ethylbenzene was 52.1 percent, with selectivity to styrene of 96.3 percent. After elimination of oxygen from the reactor with nitrogen, as in Part 2, conversion of ethylbenzene was reduced to only 2.3 percent, with 95.4 percent selectivity to styrene. Thereafter, when oxidative dehydrogenation conditions again were established, 48.1 percent conversion of ethylbenzene, with selectivity to styrene of 96.1 percent, was achieved.

## Example 5

The method of Example 1 was repeated, with Anderson AX21 carbonaceous material utilized at a bed height of 18 mm to provide a comparative example. Oxidative dehydrogenation under conditions of Part 1 of Example 1 yielded 80.0 percent conversion of ethylbenzene, with 90.1 percent selectivity to styrene. Reaction under conditions of Part 2 of Example 1 yielded 5.2 percent conversion of ethylbenzene, with 40.4 percent selectivity to styrene. Reaction in accordance with Part 3 of Example 1 was not carried out.

## Example 6

Ambersorb 563 carbonaceous adsorbent was placed in a vertical reactor, together with glass beads, as described in Example 1. The bed height (0.5 grams of catalyst) was 18 mm. Air was flowed over the catalyst bed and glass beads at a rate of 1 ml/minute as the temperature was raised to 250°C. After one hour at 250°C, a 1-butene flow of 0.5 ml/minute was mixed into the air flow.

Steady-state operation was established, as determined by consistency of selectivity and conversion, at temperatures in 50°C intervals between 250°C and 500°C. The study was interrupted after between 8 and 10 hours by interrupting the flow of 1-butene and reducing the temperature to 20°C. The pressure was about 1 atmosphere.

After 14 hours at the reduced temperature, the temperature was increased, and the temperature optimization study was continued by introducing 1-butene reactant feed. the optimum temperature for oxidative dehydrogenation of 1-butene utilizing this catalyst, as determined by maximization of 1,3-butadiene yield, was achieved at a temperature of 350°C. At this temperature, the conversion was 50 percent, with a 1,3-butadiene selectivity of 88 percent. The remaining products were lower hydrocarbons.

## Example 7

The method of Example 6 was followed with Ambersorb 575 carbonaceous adsorbent at a bed height of 15 mm for the 0.5-gram bed. At an operating pressure of about 1 atmosphere, the optimum temperature was determined to be 375°C, at which the conversion of 1-butene was 39 percent, with a selectivity to 1,3-butadiene of 81 percent.

## Example 8

The method of Example 6 essentially was repeated, with 0.5 grams of Ambersorb 564 carbonaceous adsorbent at a bed height of 12 mm. The reaction was carried out isothermally at 350°C, rather than at different temperatures. The conversion of 1-butene was 61 percent, with 82 percent selectivity to 1,3-butadiene.

## Example 9

Ambersorb 563 carbonaceous adsorbent was doped with chromium oxide in accordance with the following method. Five grams of catalyst were placed in a beaker to which was added 0.007 mols (0.7 grams) of $CrO_3$ dissolved in 5 mls of water. The resulting mixture had a clumpy texture, with no unabsorbed fluid visible. The water was removed from the catalyst by drying at 100°C under vacuum (about 0.1 mm Hg) for 2 hours.

One-half gram of the resulting catalyst was utilized in a vertical reactor in a bed having a height of 13 mm. Optimal temperature was determined to be 300°C, at which 78 percent conversion of 1-butene was achieved, with 99 percent selectivity to 1,3-butadiene.

## Example 10

Ambersorb 575 carbonaceous adsorbent was doped with chromium oxide in accordance with the method set forth in Example 9, and was utilized in accordance with the oxidative dehydrogenation method of Example 6. The optimum temperature was determined to be 350°C. The conversion of 1-butene was 68 percent, with 89 percent selectivity to 1,3-butadiene.

## Example 11

Anderson AX21 (2.5 grams) was doped with 0.45 grams of $MoO_3$ by refluxing the carbonaceous material with the dopant in 50 ml of water for 6 hours. The water was removed by rotary evaporation and subsequent drying in vacuum at 100°C.

One-half gram of this catalyst was placed in a vertical flow reactor, as described in Example 1. The bed height was 15 mm. Liquid ethanol was fed at a rate of 0.2 ml/hour, vaporized, and was mixed with an air flow of 300 ml/hour. Oxidative dehydrogenation was carried out at a temperature of 230°C over a 20 hour period. Conversion of the ethanol exceeded 99 percent, with 85 percent selectivity to acetaldehyde.

## Example 12

The method of Part 1 of Example 1 was repeated with 1.08 grams of Ambersorb 572 carbonaceous material as catalyst, which provided a bed height of 33 mm. After 140 hours on-stream, conversion of 93.3 percent of the ethylbenzene was achieved, with 92.2 percent selectivity to styrene.

The catalyst had become clumped together, and the weight of the catalyst after the reaction period had increased by 0.88 grams per gram of catalyst.

## Example 13

The method of Example 12 was repeated, utilizing 1,4-diethylbenzene as the hydrocarbon reactant. The feedrate of 1,4-diethylbenzene was the same as the hydrocarbon feedrate set forth in Example 1. Conversion was 41.3 percent, with 62.6 percent selectivity to p-ethylstyrene and 13.2 percent selectivity to divinylbenzene. The remainder of the products comprised benzene and xylenes.

## Example 14

The method of Example 12 was repeated at a reactor temperature of 300°C. Conversion was 38.7 percent, with selectivity to p-ethylstyrene of 57.4 percent and selectivity to 1,4-divinylbenzene of 27.2 percent.

## Example 15

Ambersorb 563 carbonaceous adsorbent was doped with potassium permanganate and sodium hydroxide in accordance with the following method. Five grams of catalyst were placed in a beaker to which was added 0.05 grams of $KMnO_4$ and 0.1 grams NaOH dissolved in 5 mls of water. The resulting mixture had a clumpy texture, with no unabsorbed fluid visible. The water was removed from the catalyst by drying at 140°C under vacuum of about 0.1 mm Hg for 4 hours.

The resultant catalyst was used in the oxidative dehydrogenation method set forth in Example 12 at a temperature of 325°C. After 140 hours on-stream, conversion of ethylbenzene was 82.7 percent, with 95.4 percent selectivity to styrene. The weight increase of the catalyst was only 0.1 grams per gram of catalyst, and only minimal clumping was observed.

## Example 16

Ambersorb 572 carbonaceous adsorbent was utilized in the fixed bed system described in Example 1 as catalyst for the oxidative dehydrogenation of isobutane. A gas mixture of 1 ml/min isobutane and 4 ml/min air was passes over one gram of catalyst, and the reaction was carried out at a temperature of 360°C.

Initial conversion of 9.0 percent was achieved with 95 percent selectivity to isobutylene. Steady-state conversion dropped to 8.0 percent and was maintained at that level; selectivity remained constant throughout.

## Example 17

The method of Example 12 was utilized to oxidatively dehydrogenate ethylbenzene over 1.0 gram of PPAN catalyst. After 24 hours, conversion of ethylbenzene was 80.1 percent, with 89.8 percent selectivity to styrene. After 50 hours, conversion had decreased to 38.9 percent, although selectivity increased to 92.3 percent.

## Example 18

Catalyst as prepared in Example 15 was utilized in the oxidative dehydrogenation of cyclohexane. One gram of catalyst was placed in a reactor as described in Example 1. A liquid feed of cyclohexane at a rate 0.2 ml/hr and a gaseous feed of air at a rate of 5 ml/min were brought into contact with the catalyst at a temperature of 360°C. Conversion of cyclohexane was 97.7 percent after 3 hours, with benzene the sole product.

## Claims

1. A method for oxidative dehydrogenation of a hydrocarbon compound to produce a more unsaturated product, said method comprising:

reacting the hydrocarbon compound and oxygen in the presence of oxidative dehydrogenation catalyst under conditions sufficient to remove hydrogen atoms from the hydrocarbon compound and form a more unsaturated product and water, said catalyst comprising carbonaceous material selected from:

(a) carbonaceous material having a selected specific surface area and a selected pore size distribution, the carbonaceous material being selected from polymeric carbons prepared by pyrolysis of resinous polymers, polymeric carbons prepared by pyrolysis of petroleum residues, polymeric carbons prepared by pyrolysis of other polymeric carbon precursors, and blends thereof;

(b) carbonaceous material doped with a redox-active transition metal moiety and selected from polymeric carbons prepared by pyrolysis of resinous polymers, polymeric carbons prepared by pyrolysis of petroleum residues, polymeric carbons prepared by pyrolysis of other polymeric carbon precursors, and blends thereof; and

(c) blends thereof.

2. A method as claimed in claim 1, wherein the hydrocarbon compound is selected from alkylated aromatics, alkanes, alkenes, oxygenates, and blends thereof.

3. A method as claimed in claim 2, wherein the hydrocarbon compound is selected from alkylated aromatics wherein the alkyl group has up to about 6 carbon atoms, straight chain, branched, and cyclic alkanes, alkenes, and oxygenates having up to about 6 carbon atoms, and blends thereof.

4. A method as claimed in any preceding claim, wherein the oxygen is supplied in air.

5. A method as claimed in any preceding claim, wherein the mol ratio of oxygen to hydrocarbon compound is from 0.1:1 to 5:1, preferably from 0.5:1 to 3:1.

6. A method as claimed in any preceding claim, wherein:
   (a) the reaction temperature is from 200°C to 550°C; and/or
   (b) the reaction pressure is from 34.48 x 10³ to 1.03 x 10⁶ Pa absolute (5 and about 150 psia); and/or
   (c) the liquid space velocity is from 0.01 to 10 hr⁻¹.

7. A method as claimed in any preceding claim, wherein the catalyst has microporosity, mesoporosity, and macroporosity, each of at least about 0.10 ml/g.

8. A method as claimed in claim 7, wherein both the macroporosity and microporosity are greater than the mesoporosity.

9. A method as claimed in claim 8, wherein the microporosity is greater than 0.2 ml/g, preferably greater than 0.3 ml/g.

10. A method as claimed in any preceding claim, wherein the catalyst has a specific surface area of at least about 250 m²/gram.

11. A method as claimed in any preceding claim, wherein the catalyst is selected from carbonaceous material having a selected specific surface area and a selected pore size distribution selected from polymeric carbons prepared by pyrolysis of resinous polymers, carbonaceous material doped with a redox-active transition metal moiety and selected from polymeric carbons prepared by pyrolysis of resinous polymers, and blends thereof.

12. A method as claimed in any preceding claim, wherein the dopant is selected from $CrO_3$, $MoO_3$, $MnO_2$, $V_2O_5$, $Ce(NO_3)_3$, $TiO_2$, and mixtures thereof.

13. In a method for oxidative dehydrogenation of a hydrocarbon compound in the presence of a carbonaceous catalyst to produce a more unsaturated compound, the improvement comprising increasing the activity or selectivity of the catalyst by doping the catalyst with a redox-active transition metal moiety.

14. A method as claimed in claim 13, wherein the moiety is selected from $CrO_3$, $MoO_3$, $MnO_2$, $V_2O_5$, $Ce(NO_3)_3$, $KMnO_4$, $KMnO_4$ and NaOH, $TiO_2$, and mixtures thereof.

15. A catalyst for the oxidative dehydrogenation of hydrocarbon compounds comprising carbonaceous material doped with a redox-active transition metal moiety and selected from polymeric carbons prepared by pyrolysis of resinous polymers, polymeric carbons prepared by pyrolysis of petroleum residues, polymeric carbons prepared by pyrolysis of other polymeric carbon precursors, and blends thereof.

16. A method as claimed in claim 15, wherein the dopant is selected from $CrO_3$, $MoO_3$, $MnO_2$, $V_2O_5$, $Ce(NO_3)_3$, $TiO_2$, and mixtures thereof.

17. A method for preventing agglomeration caused by deposition of byproducts of oxidative dehydrogenation of hydrocarbon compounds on oxidative dehydrogenation catalyst comprising carbonaceous material selected from:
   (a) carbonaceous material having a selected specific surface area and a selected pore size distribution selected from polymeric carbons prepared by pyrolysis of resinous polymers, polymeric carbons prepared by pyrolysis of petroleum residues, polymeric carbons prepared by pyrolysis of other polymeric carbon precursors, and blends thereof;
   (b) carbonaceous material doped with a redox-active transition metal moiety and selected from polymeric carbons prepared by pyrolysis of resinous polymers, polymeric carbons prepared by pyrolysis of petroleum residues, polymeric carbons prepared by pyrolysis of other polymeric carbon precursors, and blends thereof; and
   (c) blends thereof;
   said method comprising doping the catalyst with a moiety which catalyses the combustion of the deposited byproducts to carbon dioxide.

18. A method as claim in claim 17, wherein the byproduct catalytic moiety is selected from $CrO_3$, $MoO_3$, $MnO_2$, $V_2O_5$, $Ce(NO_3)_3$, $KMnO_4$ and NaOH, $TiO_2$, and mixtures thereof, preferably $KMnO_4$ and NaOH.